# EUROPÄISCHE PATENTSCHRIFT

(11) **EP 3 209 280 B1**
(45) Veröffentlichungstag und Bekanntmachung des Hinweises auf die Patenterteilung: **12.02.2020**
(21) Anmeldenummer: 15763943.6
(22) Anmeldetag: 18.09.2015
(51) Int. Cl.: A61K 8/894, A61K 8/34, A61Q 5/06, A61K 8/04

(54) **TEMPORÄRE FARBVERÄNDERUNG VON HAAREN MIT PIGMENTEN, ALKOHOLEN UND SPEZIELLEN POLYALKOXYLIERTEN SILIKONEN**
TEMPORARY CHANGING THE COLOUR OF HAIR USING PIGMENTS, ALCOHOLS AND SPECIFIC POLYALKOXYLATED SILICONS
MODIFICATION TEMPORAIRE DE LA COULEUR DES CHEVEUX AU MOYEN DE PIGMENTS, D'ALCOOLS ET DE SILICONES POLYALCOXYLÉES PARTICULIÈRES

(30) Priorität: 23.10.2014 DE 102014221536
(43) Veröffentlichungstag der Anmeldung: 30.08.2017
(73) Patentinhaber: Henkel AG & Co. KGaA, 40589 Düsseldorf (DE)
(72) Erfinder: GOUTSIS, Konstantin, 41363 Jüchen (DE); WESER, Gabriele, 41472 Neuss (DE)
(86) Internationale Anmeldenummer: PCT/EP2015/071465
(87) Internationale Veröffentlichungsnummer: WO 2016/062468

(56) Entgegenhaltungen:
- EP-A2- 0 172 713
- EP-A2- 1 362 576
- WO-A1-2004/112744
- WO-A2-2007/005958
- DE-T2- 68 915 171
- DE-U1- 29 917 773
- FR-A1- 2 921 832
- US-A1- 2009 119 851

## Beschreibung

Gegenstand der vorliegenden Anmeldung sind Mittel zur temporären Farbveränderung von keratinischen Fasern, insbesondere menschlichen Haaren, welche auf einem wässrig-alkoholischen Träger basieren und welche Farbpigmente sowie nichtionische, polyalkoxylierte Silikone beinhalten. Kennzeichnend für diese Mittel sind ihr hoher Gehalt an Alkoholen und ihr niedriger Gehalt an Fettstoffen. Ein weiterer Gegenstand dieser Anmeldung ist ein Verfahren zur Farbänderung und zum Styling der Haare, wobei ein entsprechendes Mittel auf die Haare aufgesprüht wird und die Haare gleichzeitig zur Frisur gelegt werden.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr lang anhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Im Rahmen moderner Modetrends besteht auch das Bedürfnis von Farbeffekten, die für einen kurzen Zeitraum auf dem Haar verbleiben und sich im Anschluss daran durch eine Haarwäsche wieder völlig rückstandslos von den Haaren entfernen lassen. Direktziehende Farbstoffe diffundieren mehr oder weniger stark in die Haarfaser hinein und überdauern dort mehrere Haarwäschen; zur rückstandslosen Entfernung des Farbeffektes ist diese Farbstoffklasse daher nicht gut geeignet.

Für eine kurzzeitige Farbveränderungen auf dem Haar ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor; diese Partikel lagern sich lediglich von außen an die Haarfaser an. Dort verbleiben sie bis zur nächsten Haarwäsche und lassen sich durch Shampoonieren wieder rückstandslos entfernen. Unter dem Namen Haar-Mascara sind verschiedene Produkte dieses Typs auf dem Markt erhältlich.

Da die Entfernung der Haar-Mascaras durch eine Haarwäsche möglich ist, sind sie im Regelfall als "leave-on" Produkte konzipiert. Von besonderem Vorteil ist es für den Anwender eines "leave-on" Produktes, wenn er gleichzeitig mit der temporären Farbänderung auch eine leichte temporäre Formgebung der Haare vornehmen kann. Als temporäre Formgebungen kommen beispielsweise Gestaltungen wie Lockung, Glättung, Toupierung oder auch Festigung in Frage. Temporäre Formgebungen können beispielsweise durch Stylingmittel, wie Haarsprays, Haarwachse, Haargele, Haarfestiger, Fönwellen, Stylingsprays usw. erzielt werden. Das temporäre Formgeben wird auch als Hair-Styling oder Styling bezeichnet, Formgebungsmittel werden auch als Stylingmittel bezeichnet.

Produkte, welche die gleichzeitige Farb- und Formänderung erlauben, sind bereits aus dem Stand der Technik bekannt. Beispielsweise werden in WO 99/20230 A2 Haar-Mascara Produkte beschrieben, welche Pigmente zusammen nichtionischen Polymeren und hochschmelzenden Wachse enthalten.

Auch WO 2014/146818 A1 offenbart Stylingmittel mit Pigmenten, die sich durch die Anwesenheit von festen Fettalkoholen und Wachsen auszeichnen.

WO 2004/112744 A1 beschreibt Polyol-in-Silikon Emulisonen.

DE 29917773 U1 betrifft verschäumbare, farbige Gele, die Pigmente zum temporären Zweck der Färbung enthalten.

Die in diesen Produkten enthaltenen Fett- und Wachsbestandteile dienen meist der Einstellung einer bestimmten Trocknungszeit, durch die der Konsument zwar das Gefühl trockener Haare erfährt, der auf das Haar aufgetragene Mascara aber eine Restfeuchte beibehält, durch welche die Frisur formbar und kämmbar bleibt.

Gerade bei auf den Haaren verbleibenden Produkten besteht jedoch oft auch das Problem, dass die Haare durch die Anwesenheit der Fettkörper beschwert werden. Visuell entsteht der Eindruck von "fettigem Haar", und die festigenden Eigenschaften dieser Produkte sind vergleichsweise schlecht. Bei Kombinationsprodukten zur Farbänderung und zum Styling besteht daher weiterhin noch Verbesserungspotential.

Die Aufgabe der vorliegenden Erfindung bestand in der Bereitstellung eines vielfältig einsetzbaren Haar-Mascara Produktes, welches die temporäre Farbänderung von Haaren ermöglicht. Das Haar-Mascara sollte so konfektionierbar sein, dass es mittels eines Schwamms, über eine Bürste und auch auf dem Wege einer Sprühapplikation aufgetragen werden kann. Die Farbänderung sollte hierbei einfach und schädigungsarm erfolgen und durch eine Wäsche auch wieder rückstandlos von den Haaren entfernbar sein. Bis zum Zeitraum der nächsten Haarwäsche sollte das auf den Haaren befindliche Produkt gegenüber äußeren Einflüssen jedoch äußerst resistent sein, d.h. weder durch Abrieb auf Textilien noch durch Kämmen sollte ein Farbverlust oder eine sonstige Ablösung des Produktes sichtbar werden. Gleichzeitig sollte das auf diese Weise farbveränderte Haar einen weichen Griff aufweisen, nicht beschwert sein, sich nicht hart oder fettig anfühlen und auch visuell nicht den Eindruck von fettigen Haaren hinterlassen.

Überaschenderweise hat sich gezeigt, dass diese Aufgaben durch Einsatz von Farbpigmenten und bestimmten nichtionischen, polyethoxylierten Silikonen gelöst werden kann, wenn diese in einem speziellen wässrig-alkoholischen Träger eingesetzt werden, der sich durch einen hohen Gehalt an Alkoholen und einen niedrigen Gehalt an Fettkörpern auszeichnet.

Ein erster Gegenstand der vorliegenden Erfindung ist ein Mittel zur temporären Farbveränderung von keratinischen Fasern, insbesondere menschlichen Haaren, enthaltend in einem wässrigen kosmetischen Träger - bezogen auf das Gesamtgewicht des Mittels -
(a) einen oder mehrere aliphatische und/oder aromatische Alkohole mit 2 bis 8 C-Atomen in einer Gesamtmenge von mindestens 35,0 Gew.-%,,
(b) mindestens ein Farbpigment, das bei 20 °C in Wasser eine Löslichkeit von weniger als 0,1 g/l besitzt, und
(c) mindestens ein nichtionisches, polyalkoxyliertes Silikon, welches mindestens eine Struktureinheit der allgemeinen Formel (II) umfasst, 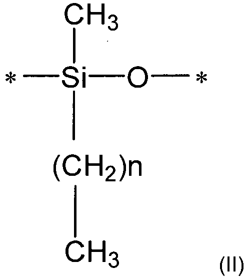 wobei
   n für eine ganze Zahl von 8 bis 40 steht, und wobei
   - die Gesamtmenge der im Mittel enthaltenen Fettstoffe (d) aus der Gruppe der C₁₂-C₃₀-Fettalkohole, der C₁₂-C₃₀-Fettsäuretriglyceride, C₁₂-C₃₀-Fettsäurediglyceride, der C₁₂-C₃₀-Fettsäuremono-glyceride, der Ethylenglycol-Difettsäureester, der Wachse und der Kohlenwasserstoffe bei einem Wert unterhalb von 2,5 Gew.-% liegt, und
   - das Mittel mindestens 30,0 Gew.-% Ethanol enthält.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Der Begriff "temporäre Farbveränderung" wird im Rahmen dieser Erfindung für eine temporäre Farbgebung des Haares verstanden, die sich durch eine Haarwäsche (mit einem handelsüblichen Shampoo) vollständig oder nahezu vollständig entfernen lässt. Nicht unter den Begriff temporäre Farbveränderung im Sinne der vorliegenden Erfindung fällt eine mit Oxidationsfarbstoffen vorgenommene oxidative Färbung. Ebenfalls nicht unter den Begriff temporäre Farbveränderung fällt eine durch Anwendung eines Oxidationsmittels hervorgerufene Aufhellung, Bleiche oder Blondierung der Keratinfasern. Sowohl der durch die oxidative Farbänderung und der durch die Blondierung hervorgerufene Effekt lässt sich durch eine Haarwäsche nicht wieder rückgängig machen, beide Farbänderungen sind daher nicht temporär.

Die Mittel enthalten die erfindungswesentlichen Inhaltsstoffe jeweils in einem wässrigen kosmetischen Träger. Zum Zwecke der temporären Farb- und Formänderung können solche Träger beispielsweise Gele oder auch tensidhaltige schäumende Lösungen, wie beispielsweise Shampoos, sprühbare Lösungen, Schaumaerosole oder Schaumformulierungen sein.

Als ersten erfindungswesentlichen Inhaltsstoff (a) enthalten die erfindungsgemäßen Mittel einen oder mehrere aliphatische und/oder aromatische Alkohole mit 2 bis 8 C-Atomen in einer Gesamtmenge von mindestens 35,0 Gew.-%.

Hierunter wird erfindungsgemäß verstanden, dass die erfindungsgemäßen Mittel einen oder mehrere aliphatische und/oder aromatische Alkohole (a) mit 2 bis 8 C-Atome in einer Gesamtmenge von mindestens 35,0 Gew.-% enthalten.

Aliphatische und/oder aromatische Alkohole mit 2 bis 8 C-Atomen sind Verbindungen, welche 2 bis 8 C-Atome besitzen, aliphatischer und/oder aromatischer Natur sind und eine oder mehrere Hydroxygruppen tragen.

Die Alkohole (a) im Sinne der vorliegenden Erfindung tragen keine von Sauerstoff unterschiedlichen Heteroatome. Sie können eine Ethergruppierung beinhalten, besitzen darüber hinaus jedoch keine funktionellen Gruppen, die von der Hydroxygruppe-Gruppe unterschiedlich sind (d.h. Monoethanolamin, alpha-Hydroxycarbonsäuren, Dihydroxyaceton usw. sind keine Alkohole im Sinne der vorliegenden Erfindung).

Geeignete aliphatische Alkohole sind beispielsweise Ethanol, Isopropanol, n-Propanol, Butanol, n-Pentanol, 1,2-Propandiol, 1,3-Propandiol, 1,3-Butandiol, 1,4-Butandiol, 1,2-Hexandiol, 1,6-Hexandiol und Glycerin. Geeignete aromatische Alkohole sind beispielsweise Benzylalkohol, Phenoxyethanol und Phenylethylalkohol.

In einer Ausführungsform ist ein erfindungsgemäßes Mittel zur temporären Farbänderung von keratinischen Fasern dadurch gekennzeichnet, dass es einen oder mehrere Alkohole (a) aus der Gruppe Ethanol, Isopropanol, n-Propanol, Butanol, n-Pentanol, 1,2-Propandiol, 1,3-Propandiol, 1,3-Butandiol, 1,4-Butandiol, 1,2-Hexandiol, 1,6-Hexandiol, Glycerin, Benzylalkohol, Phenoxyethanol und Phenylethylalkohol enthält.

Bei den erfindungsgemäßen Alkoholen (a) handelt es sich um organische Lösungsmittel, die zur Lösung des nichtionischen polyalkoxylierten Silikons (c) beitragen und nach Auftragung des Mittels auf die keratinische Faser die Geschwindigkeit der Filmbildung der Silikone (c) beeinflussen. Der bzw. die Alkohole (a) sind in einer Mindestmenge von 35,0 Gew.-% im erfindungsgemäßen Mittel enthalten.

Es hat sich herausgestellt, dass diese Filmbildung dann besonders gut abläuft und besonders gleichmäßig ist, wenn der oder die Alkohole in einer Mindestmenge von mindestens 35,0 Gew.-%, noch weiter bevorzugt von mindestens 40,0 Gew.-% und ganz besonders bevorzugt von mindestens 50,0 Gew.-% im Mittel enthalten sind. Die besten Ergebnisse wurden bei einer Alkoholmenge von mindestens 40 Gew.-% beobachtet. Alle Mengenangaben in Gew.-% sind hierbei auf die Gesamtmenge aller erfindungsgemäßen Alkohole (a) bezogen, die zum Gesamtgewicht des Mittels in Relation gesetzt wird.

In einer bevorzugten Ausführungsform ist ein erfindungsgemäßes Mittel dadurch gekennzeichnet, dass es - bezogen auf sein Gesamtgewicht - einen oder mehrere Alkohole (a) in einer Gesamtmenge von mindestens 40,0 Gew.-% und ganz besonders bevorzugt von mindestens 50,0 Gew.-% enthält
Bei den Alkoholen aus der Gruppe (a) handelt es sich um Verbindungen, die verschieden hohe Siedepunkte besitzen und verschieden stark flüchtig sind. Es hat sich herausgestellt, dass Ethanol innerhalb dieser Gruppe die beste Eignung besitzt. Wenn die Mittel signifikante Mengen an Ethanol enthalten, werden die auf der Keratinfaser abgelagerten Pigmente von einem Polymerfilm der Sillikone (c) umschlossen, der so ausgebildet ist, dass die Pigmente besonders gut auf der Keratinfaser haften. In diesem Fall ist das Farbergebnis besonders gleichmäßig und der durch Reibung an Textilien hervorgerufene Abrieb der Pigmente minimiert.

Ein erfindungsgemäßes Mittel ist daher dadurch gekennzeichnet, dass es - bezogen auf sein Gesamtgewicht - mindestens 30,0 Gew.-%, bevorzugt mindestens 35,0 Gew.-%, weiter bevorzugt mindestens 40,0 Gew.-% und ganz besonders bevorzugt mindestens 45,0 Gew.-% Ethanol enthält. Noch weiter verbessert werden können die zuvor beschriebenen Eigenschaften, wenn dem Ethanol in geringerer Menge ein weiterer mehrwertiger Alkohol mit niedrigerer Flüchtigkeit, beispielsweise 1,2-Propandiol oder Glycerin, zugesetzt wird.

In einer weiteren ganz besonders bevorzugten Ausführungsform ist ein erfindungsgemäßes Mittel daher dadurch gekennzeichnet, dass es - bezogen auf sein Gesamtgewicht - 1,2-Propandiol und/oder Glycerin in einer Gesamtmenge von 0,1 bis 7,0 Gew.-%, bevorzugt 0,5 bis 5,5 Gew.-%, weiter bevorzugt 1,0 bis 3,5 Gew.-% und besonders bevorzugt 1,5 Gew.-% bis 2,5 Gew.-% enthält. Die erfindungsgemäßen Mittel enthalten alle wesentlichen Bestandteile in einem wässrigen Träger. Durch den Wassergehalt des Mittels lasst sich die Ablagerung der Pigmente auf den Keratinfasern und die Filmbildung der Silikone (c) ebenfalls beeinflussen. Ist der Wassergehalt zu hoch, besteht die Gefahr, dass das Produkt nicht ausreichend schnell trocknet. Insbesondere wenn die Mittel auf eine niedrigere Viskosität eingestellt werden (beispielsweise weil sie versprüht werden sollen), kann dass das Farbergebnis dann ungleichmäßiger ausfallen. Als gut geeignet hat sich in diesem Zusammenhang ein Wassergehalt zwischen 20 und 60 Gew.-%, bevorzugt zwischen 24 und 54 Gew.-%, weiter bevorzugt zwischen 28 und 50 Gew.-% und besonders bevorzugt zwischen 32 und 42 Gew.-% herausgestellt. Der in Gew.-% angegebene Wassergehalt bezieht sich hierbei auf die Menge an Wasser, die im Gesamtgewicht des Mittels enthalten ist.

In einer weiteren ganz besonders bevorzugten Ausführungsform ist ein erfindungsgemäßes Mittel zur temporären Farbveränderung daher dadurch gekennzeichnet, dass es - bezogen auf sein Gesamtgewicht - einen Wassergehalt zwischen 20 und 60 Gew.-%, bevorzugt zwischen 24 und 54 Gew.-%, weiter bevorzugt zwischen 28 und 50 Gew.-% und besonders bevorzugt zwischen 32 und 42 Gew.-% besitzt.

Als zweiten erfindungswesentlichen Bestandteil enthalten die Mittel zur temporären Farbveränderung mindestens ein Farbpigment (b). Unter einem Pigment im Sinne der vorliegenden Erfindung wird eine farbgebende Verbindung verstanden, die bei 20 °C in Wasser eine Löslichkeit von weniger als 0,1 g/l besitzt.

Die folgende Methode lässt sich zur Bestimmung der Wasserlöslichkeit des Pigments anwenden: 0,1 g des Pigments werden in einem Becherglas abgewogen. Ein Rührfisch wird hinzugefügt. Dann wird mit destilliertem Wasser (20 °C) auf 1 l aufgefüllt. Es wird für eine Stunde gerührt. Sind in der Mischung nach diesem Zeitraum noch ungelöste Bestandteile des Pigments sichtbar, so liegt die Löslichkeit des Pigments unterhalb von 0,1 g/l.

Mit den erfindungsgemäßen Mitteln soll eine temporäre Farbgebung erfolgen. Im Vordergrund steht hierbei insbesondere die Erzeugung von "metallic" Effekten. Nicht unter die Definition der Farbpigmente fallen daher die Weißpigmente. Weißpigmente sind unbunte anorganische Pigmente mit einem hohen Brechungsindex (idR größer 1,8), die in der Regel synthetisch hergestellt und vor allem zur Erzeugung von optischer Weiße in Anstrichmitteln oder als Füllstoff in z. B. Kunststoffen verwendet werden. Weißpigmente wie beispielsweise Titandioxid oder Zinkdioxid sind von der Definition eines Farbpigments explizit nicht mit umfasst.

In den Mitteln liegen die Farbpigmente in Form von kleinen ungelösten Partikeln vor, die nicht in die Haarfaser hineindiffundieren, sondern sich unter Einfluss des Silikons (c) auf der Außenwand der Keratinfaser ablagern und dort durch eine polymere Silikonschicht gehalten werden.

Geeignete Farbpigmente können organischen und/oder anorganischen Ursprungs sein.

Aufgrund ihrer ausgezeichneten Licht-, Wetter- und/oder Temperaturbeständigkeit ist die Verwendung anorganischer Farbpigmente in dem erfindungsgemäßen Verfahren besonders bevorzugt. Die bevorzugte mittlere Teilchengröße der - bevorzugt anorganischen - Farbpigmente beträgt 0,1 µm bis 1 mm, mehr bevorzugt von 0,5 µm bis 750 µm und insbesondere 10 µm bis 500 µm.

Bevorzugte Farbpigmente sind ausgewählt aus anorganischen Pigmenten, die synthetischen oder natürlichen Ursprungs sein können. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß besonders bevorzugte Farbigmente sind farbige Perlglanzpigmente. Diese basieren üblicherweise auf Mica- und/oder Glimmerbasis und können mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sein.

Glimmer gehören zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehreren Metalloxid(en) beschichtetes, synthetisches Mica als Perlglanzpigment verwendet werden. Solche geeigneten Perlglanzpigmente auf der Basis natürlichen Micas werden in der Offenlegungsschrift WO 2005/065632 beschrieben, auf die ausdrücklich Bezug genommen wird. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren besonders bevorzugten Ausführungsform ist ein erfindungsgemäßes Mittel dadurch gekennzeichnet, dass es als Farbpigment (b) mindestens ein anorganisches Farbpigment enthält, welches ausgewählt ist aus farbigen Metalloxiden, Metallhydroxiden, Metalloxidhydraten, Silicaten, Metallsufiden, komplexen Metallcyaniden, Metallsulfaten, Bronzepigmenten und/oder aus farbigen Pigmenten auf Mica- oder Glimmerbasis, die mit mindestens einem Metalloxid und/oder einem Metalloxychlorid beschichtet sind.

In einer weiteren ganz besonders bevorzugten Ausführungsform ist ein erfindungsgemäßes Mittel dadurch gekennzeichnet, dass es als Farbpigment (b) mindestens ein farbiges Pigment auf Mica- oder Glimmerbasis enthält, das mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet ist.

Beispiele für besonders geeignete Farbpigmente sind im Handel beispielsweise unter den Handelsbezeichnungen Rona®, Colorona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich. Ganz besonders bevorzugte Farbigmente mit der Handelsbezeichnung Colorona® sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM
DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 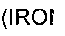
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)

Weiterhin besonders bevorzugte Farbpigmente mit der Handelsbezeichnung Unipure® sind beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Abhängig davon, welche Farbveränderung auf der Kertinfaser erwünscht wird, können das oder die Farbpigmente (b) in unterschiedlichen Mengen eingesetzt werden. Je mehr Pigment eingesetzt wird, desto höher ist generell das Ausmaß der Farbveränderung. Ab einer bestimmten Einsatzmenge läuft jedoch die Haftung der Pigmente an der Keratinfaser gegen einen Grenzwert, ab welchem es nicht mehr möglich ist, das Ausmaß der Farbveränderung durch weitere Erhöhung der eingesetzten Pigmentmenge zu steigern.

In diesem Zusammenhang hat sich herausgestellt, dass bei Einsatz der erfindungsgemäßen Silikone (c) - insbesondere der genannten bevorzugten und besonders bevorzugten Vertreter - auf der Keratinfaser ein Film ausgebildet werden kann, der die Pigmente in besonders großen Mengen auf der Keratinfaser haften lässt. Die erfindungsgemäßen Mittel können die Farbpigmente (b) daher in einer Gesamtmenge von 1,0 bis 25,0 Gew-%, bevorzugt von 5,0 bis 20,0 Gew.-%, weiter bevorzugt von 7,0 bis 18,0 Gew.-% und besonders bevorzugt von 8,5 bis 15,5 Gew.-% enthalten.

In einer weiteren besonders bevorzugten Ausführungsform ist ein erfindungsgemäßen Mittel daher dadurch gekennzeichnet, dass es - bezogen auf sein Gesamtgewicht - ein oder mehrere Farbpigmente (b) in einer Gesamtmenge von 1,0 bis 25,0 Gew-%, bevorzugt von 5,0 bis 20,0 Gew.-%, weiter bevorzugt von 7,0 bis 18,0 Gew.-% und besonders bevorzugt von 8,5 bis 15,5 Gew.-% enthält.

Als dritten erfindungswesentlichen Bestandteil (c) enthalten die Mittel zur temporären Farbveränderung von Keratinfasern mindestens ein nichtionisches, polyalkoxyliertes Silikon, welches mindestens eine Struktureinheit der allgemeinen Formel (II) umfasst, 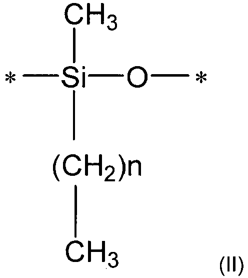 wobei
n für eine ganze Zahl von 8 bis 40 steht.

Silikone sind polymere Verbindungen, die nach dem Schema (R2SiO)x aufgebaut sind, wobei R üblicherweise für einen organischen Rest, oft eine Alkylgruppe oder auch eine substituierte Alkylgruppe, steht. Silikone werden auch als Polyorganosiloxane bezeichnet, sie können linear oder verzweigt sein. Verknüpft sind die Siliciumatome über Sauerstoffatome in einer Si-O-Si-Bindung. Polyalkoxylierte Silikone tragen als Struktureinheiten Polyoxyalkylengruppen, insbesondere Polyoxyethylengruppen (d.h. Gruppen des Typs [-CH2-CH2-O-]ₘ) und/oder Poloxypropylengruppen (d.h. Gruppen des Typs [-CH(CH3)-CH2-O-]ₘ und/oder [-CH2-CH2-CH2-O-]ₘ). Die Zahl m der Polyoxyalkyleineinheiten im Silikon-Polymer liegt bei mindestens 2.

Unter polymeren Verbindungen werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Das maximale Molekulargewicht des Silikons hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikons (c) nicht mehr als 10⁷ g/mol, bevorzugt nicht mehr als 10⁶ g/mol und besonders bevorzugt nicht mehr als 10⁵ g/mol beträgt.

Bei den erfindungsgemäßen polyalkoxylierten Silikonen handelt es sich um nichtionische Verbindungen, die Silikone tragen demnach weder positive noch negative Ladungen.

Die erfindungsgemäßen nichtionischen Silikone umfassen Struktureinheiten der Formel (a)

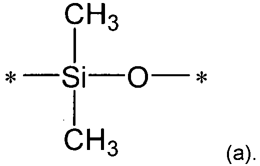

Die Struktureinheiten der Formel (a) stellen die Wiederholungseinheiten des Silikons dar, die Verknüpfung von zwei Struktureinheiten der Formel (a) führt beispielsweise zur Formel (a')

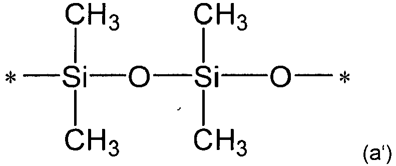

Weiterhin sind die erfindungsgemäßen Silikone polyalkoxyliert und umfassen daher mindestens zwei Alkylenoxid-Einheiten. In einer bevorzugten Ausführungsform sind diese Alkylenoxid-Einheiten über eine Alkylengruppe, besonders bevorzugt über eine n-Propylengruppe, mit einem Silicium-Atom verbunden. Bevorzugte Silikone (c) umfassen demnach mindestens eine Struktureinheit der allgemeinen Formel (I)

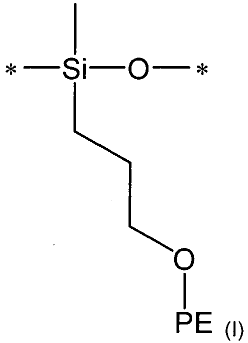

Die Gruppierung PE steht hierbei für eine Gruppierung -(C₂H₄O)ₓ-(C₃H₆O)_{y}-H oder für eine Gruppierung -(C₃H₆O)_{y}-(C₂H₄O)ₓ-H, x steht für eine ganze Zahl von 0 bis 20 und y steht für eine ganze Zahl von 0 bis 20, wobei die Summe aus x und y mindestens 2 beträgt. In einer weiteren ganz besonders bevorzugten Ausführungsform ist ein erfindungsgemäßes Mittel dadurch gekennzeichnet, dass es ein nichtionisches, polyalkoxyliertes Silikon (c) enthält, welches mindestens eine Struktureinheit der allgemeinen Formel (I) umfasst, 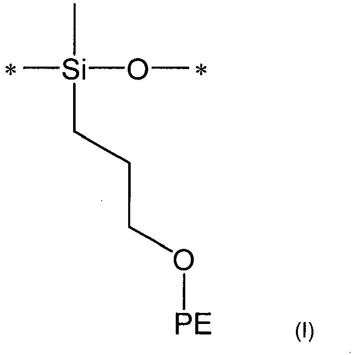 wobei
PE gleich für eine Gruppierung -(C₂H₄O)ₓ-(C₃H₆O)_{y}-H oder für eine Gruppierung -(C₃H₆O)_{y}-(C₂H₄O)ₓ-H steht,
x für eine ganze Zahl von 0 bis 20 steht,
y für eine ganze Zahl von 0 bis 20 steht und
die Summe aus x und y mindestens 2 beträgt.

Die Summe aus x und y beträgt mindestens 2. Steht x für 1, dann steht y für eine ganze Zahl von 1 bis 20. Steht x für 2 oder für eine für ganze Zahl von mehr als 2, dann kann y auch für 0 stehen. Steht y für 2 oder für eine ganze Zahl von mehr als 2, dann kann x auch für 0 stehen.

Besonders bevorzugte Silikone (c) umfassen beispielsweise die folgenden Struktureinheiten: 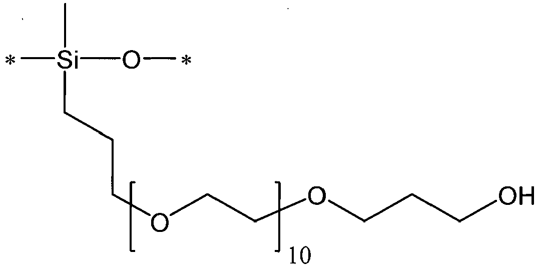 und/oder

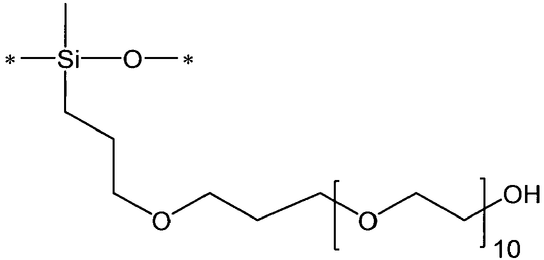

In einer weiteren ganz besonders bevorzugten Ausführungsform ist ein erfindungsgemäßes Mittel dadurch gekennzeichnet, dass es ein nichtionisches, polyalkoxyliertes Silikon (c) enthält, welches mindestens eine Struktureinheit der allgemeinen Formel (la) umfasst, 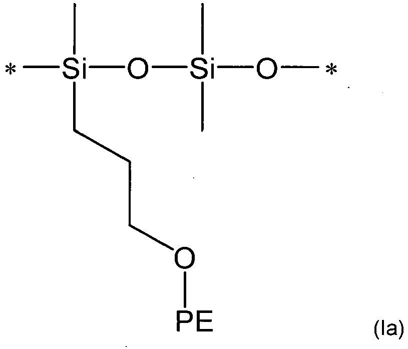 wobei
PE gleich für eine Gruppierung -(C₂H₄O)ₓ-(C₃H₆O)_{y}-H oder für eine Gruppierung -(C₃H₆O)_{y}-(C₂H₄O)ₓ-H steht,
x für eine ganze Zahl von 0 bis 20 steht,
y für eine ganze Zahl von 0 bis 20 steht und
die Summe aus x und y mindestens 2 beträgt.

In einer bevorzugten Ausführungsform steht x für eine ganze Zahl von 8 bis 12, und y steht für die Zahlen 0 oder 1.
In einer ganz besonders bevorzugten Ausführungsform steht x für die Zahl 10, und y steht für die Zahl 1.

Besonders gut geeignete Silikone sind beispielsweise:
Abil EM 90, der Firma Evonic , CETYL PEG/PPG-10/1 DIMETHICONE
Microcare Silicone E 1016 der Firma Thor (Parka), Cetyl PEG/PPG-10/1 Dimethicone

Die erfindungsgemäßen nichtionischen Silikone (c) bilden auf den Keratinfasern Filme, welche die Farbpigmente (b) auf der Keratinfasern einschließen und diese so gegen Abrieb schützen. Hierbei hat sich herausgestellt, dass insbesondere die polalkoxylierten Silikone zur Ausfildung von stabilen Filmen gut geeignet sind, die als zusätzliche Struktureinheit eine C8-C40 Alkylgruppe tragen.
Ein erfindungsgemäßes Mittel ist dadurch gekennzeichnet, dass es ein nichtionisches, polyalkoxyliertes Silikon (c) enthält, welches mindestens eine Struktureinheit der allgemeinen Formel (II) umfasst, 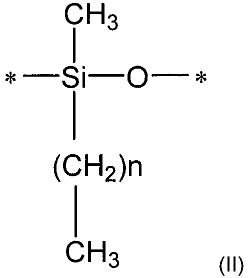 wobei
n für eine ganze Zahl von 8 bis 40, bevorzugt von 10 bis 30 und besonders bevorzugt von 14 bis 20 steht.

Die mit einem Stern gekennzeichneten Positionen können hierbei jeweils die Verknüpfungsstelle zu einer anderen Siloxaneinheit darstellen. Die Struktureinheit der Formel (II) kann das Silikon jedoch auch terminieren. In diesem Fall stellt die Struktureinheit der Formel (II) die Endgruppe des Silikons dar, d.h. eine mit einem Stern gekennzeichnete Position stellt die Verknüpfungsstelle zu einer anderen Siloxaneinheit dar, wohingegen die andere mit einem Stern gekennzeichnete Position für die Bindung zu einer Methylgruppe, einer Methoxygruppe oder einer Hydroxygruppe stehen kann Formulierungen, die ein nichtionisches polyalkoxyliertes Silikon mit mindestens einer Struktureinheit der Formel (I) und mindestens einer Struktureinheit der Formel (II) enthalten, weisen eine sehr feindisperse Verteilung der Farbpigmente (b) in der alkoholisch-wässrigen Lösung auf. Die Formulierungen sind sehr gut sprühbar, und die Pigmente lassen sich gleichmäßig auf den Keratinfasern verteilen, so dass ein besonders homogenes Farbergebnis erzielt werden kann.

Die besten Ergebnisse ließen sich mit CETYL PEG/PPG-10/1 DIMETHICONE erzielen.

In einer ganz besonders bevorzugten Ausführungsform ist ein erfindungsgemäßes Mittel daher dadurch gekennzeichnet, dass als nichtionisches, polyalkoxyliertes Silikon (c) Cetyl PEG/PPG-10/1 Dimethicon enthält.

Die erfindungsgemäßen Mittel enthalten das bzw. die Silikone (c) üblicherweise in einer Gesamtmenge von 0,1 bis 10,0 Gew-%, bevorzugt von 0,5 bis 5,0 Gew.-%, weiter bevorzugt von 0,8 bis 3,0 Gew.-% und besonders bevorzugt von 1,4 bis 2,6 Gew.-%. Hierbei beziehen sich die Mengenangaben in Gew.-% auf die Gesamtmenge aller nichtionischen, polyalkoxylierten Silikone (c), die zum Gesamtgewicht des Mittels in Relation gesetzt werden.

In einer weiteren besonders bevorzugten Ausführungsform ist ein erfindungsgemäßes Mittel dadurch gekennzeichnet, dass es - bezogen auf sein Gesamtgewicht - ein oder mehrere nichtionische, polyalkoxylierte Silikone (c) in einer Gesamtmenge von 0,1 bis 10,0 Gew-%, bevorzugt von 0,5 bis 5,0 Gew.-%, weiter bevorzugt von 0,8 bis 3,0 Gew.-% und besonders bevorzugt von 1,4 bis 2,6 Gew.-% enthält.

Für ein optimales Haftungsvermögen der Farbpigmente (b) an den Keratinfasern werden die Einsatzmengen von Pigmenten (b) und Silikonen (c) vorteilhafterweise aufeinander abgestimmt. Werden Pigmente (b) und Silikone (c) in einem Gewichtsverhältnis von 1,0 bis 6,0 eingesetzt, so kann der Großteil der Pigmente effektiv über den Silikonfilm gebunden und auf diese Weise an der Faser immobilisiert werden. Mit anderen Worten ist es von besonderem Vorteil, Farbpigmente (b) und Silikone (c) mindestens in gleichen Gesamtmengen einzusetzen, oder aber Einsatzmengen zu wählen, bei denen die Gesamtmenge der Farbpigmente (b) die Gesamtmenge der Silikone (c) höchstens um den Faktor 6 übersteigt. Bei dem angegebenen Gewichtsverhältnis (b)/(c) wird die im Mittel enthaltene Gesamtmenge der Pigmente (b) zu der im Mittel enthaltenen Gesamtmenge an Silikonen (c) in Relation gesetzt.

In einer weiteren ganz besonders bevorzugten Ausführungsform ist ein erfindungsgemäßes Mittel dadurch gekennzeichnet, dass das Gewichtsverhältnis aus allen im Mittel enthaltenden Farbpigmenten (b) zu allen im Mittel enthaltenen Silikonen (c), d.h. das Gewichtsverhältnis (b)/(c), bei einem Wert von 1,0 bis 6,0, bevorzugt von 2,0 bis 5,5, weiter bevorzugt von 2,5 bis 5,0 und besonders bevorzugt von 3,0 bis 4,5 liegt.

### Beispiel: ein temporäres Färbemittel enthält

35,0 Gew.-% Wasser
   (a) 40,0 Gew.-% Ethanol
   (b) 8,0 Gew.-% Colorona Bronze, Merck, MICA, CI 77491 (IRON OXIDES)
   (c) 2,0 Gew.-% Cetyl PEG/PPG-10/1 Dimethicone
weitere Inhaltsstoffe ad 100 Gew.-%
Gewichtsverhältnis (b)/(c) = 4,0

Die aus dem Stand der Technik bekannten Haar-Mascara Produkte enthalten in aller Regel Fettstoffe; diese Fettstoffe bilden einen Film auf den Keratinfasern aus, welche die Pigmente nach der Anwendung vor dem Abrieb schützt.

Der wesentliche Nachteil der Fettstoffe ist jedoch, dass sie auf der Keratinfaser eine wenig vorteilhaften Haptik erzeugen, die sich insbesondere in einem Gefühl der Härte und des fettigen Haargefühls äußert. Die Keratinfasern wirken beschwert und vermitteln auch visuell den Eindruck von fettigem Haar.

Zur Vermeidung dieses Nachteils ist es ein kennzeichnendes und wesentliches Merkmal der erfindungsgemäßen Mittel, dass die Gesamtmenge der im Mittel enthaltenen Fettstoffe (d) aus der Gruppe der C₁₂-C₃₀-Fettalkohole, der C₁₂-C₃₀-Fettsäuretriglyceride, C₁₂-C₃₀-Fettsäurediglyceride, der C₁₂-C₃₀-Fettsäuremonoglyceride, der Ethylenglycol-Difettsäureester, der Wachse und der Kohlenwasserstoffe - bezogen auf das Gesamtgewicht des Mittels - bei einem Wert unterhalb von 2,5 Gew.-% liegt.

Unter "Fettstoffen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Geladene Verbindungen wie beispielsweise Fettsäuren und ihre Salze werden nicht als Fettbestandteil verstanden. Fettstoffe im Sinne der vorliegenden Erfindung besitzen mindestens eine gesättigte oder ungesättigte Alkylgruppe mit mindestens 12 C-Atomen. Enthalten die Fettstoffe eine ungesättigte Alkylgruppe, so kann diese eine oder mehrere Doppelbindungen besitzen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen, d.h. Fettalkohole oder Fettsäuren, die mit mindestens zwei Oxyalkylgruppen bzw. mit mindestens zwei Glycerineinheiten verestert oder verethert sind, fallen nicht unter die Definition der Fettstoffe.

Unter die Fettstoffe (d) fallen C₁₂-C₃₀-Fettalkohole. C₁₂-C₃₀-Fettalkoholen sind gesättigte, ein- oder mehrfach ungesättigte, lineare oder verzweigte Fettalkohole mit 12 bis 30 C-Atomen handeln. Beispiele für C₁₂-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol). Beispiele für verzweigte Fettalkohole sind 2-Octyl-dodecanol, 2-Hexyl-Dodecanol und/oder 2-Butyl-dodecanol. Unter die Fettstoffe (d) fallen auch C₁₂-C₃₀-Fettsäuretriglyceride. Unter einem C₁₂-C₃₀-Fettsäuretriglycerid wird der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein. Unter die Fettstoffe fallen auch C12-C30-Fettsäurediglyceride. Unter einem C₁₂-C₃₀-Fettsäurediglycerid wird der Diester des dreiwertigen Alkohols Glycerin mit zwei Äquivalenten Fettsäure verstanden. Dabei können sowohl strukturgleiche als auch unterschiedliche Fettsäuren innerhalb eines Diglyceridmoleküls an den Esterbildungen beteiligt sein. Unter die Fettstoffe fallen auch C12-C30-Fettsäuremonoglyceride. Unter einem C12-C30-Fettsäuremonoglycerid wird der Monoester des dreiwertigen Alkohole Glyceirn mit einem Äquivalent Fettsäure verstanden.

Unter die Fettstoffe (d) fallen auch die Diester aus einem Äquivalent Ethylenglycol (1,2-Ethandiol) mit zwei Äquivalenten Fettsäure (Ethylenglycol-Difettsäureester). Dabei können sowohl strukturgleiche als auch unterschiedliche Fettsäuren an den Esterbindungen mit dem Ethylenglycol beteiligt sein.

Unter die Fettstoffe (d) fallen auch Wachse. Unter Wachsen werden die Ester von C12-C30-Fettsäuren mit C12-C30-Fettalkoholen verstanden.

Unter die Fettstoffe (d) fallen auch Kohlenwasserstoffe mit mindestens 12 C-Atomen. Kohlenwasserstoffe sind ausschließlich aus den Atomen Kohlenstoff und Wasserstoff bestehende Verbindungen. Beispiele für Kohlenwasserstoffe sind Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Silikone sind von der Definition der Fettstoffe nicht umfasst.

Erfindungsgemäß ist demnach ein Mittel zur temporären Farbveränderung von keratinischen Fasern, insbesondere menschlichen Haaren, enthaltend in einem wässrigen kosmetischen Träger- bezogen auf sein Gesamtgewicht -
(a) einen oder mehrere aliphatische und/oder aromatische Alkohole mit 2 bis 8 C-Atomen in einer Gesamtmenge von mindestens 35,0 Gew.-%
(b) mindestens ein Farbpigment das bei 20 °C in Wasser eine Löslichkeit von weniger als 0,1 g/l besitzt, und
(c) mindestens ein nichtionisches, polyalkoxyliertes Silikon welches mindestens eine Struktureinheit der allgemeinen Formel (II) umfasst, 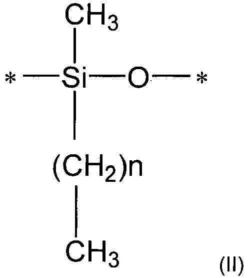 wobei
   n für eine ganze Zahl von 8 bis 40 steht, und wobei
   - die Gesamtmenge der im Mittel enthaltenen Fettstoffe (d) aus der Gruppe der C₁₂-C₃₀-Fettalkohole, der C₁₂-C₃₀-Fettsäuretriglyceride, C₁₂-C₃₀-Fettsäurediglyceride, der C₁₂-C₃₀-Fettsäuremonoglyceride, der Ethylenglycol-Difettsäureester, der Wachse und der Kohlenwasserstoffe bei einem Wert unterhalb von 2,5 Gew.-% liegt, und
   - das Mittel mindestens 30,0 Gew.-% Ethanol enthält.

Durch Einsatz bestimmter Rohstoffe können unter Umständen geringe Mengen an Fettstoffen in die erfindungsgemäßen Mittel eingeschleppt werden. Um das Haar möglichst wenig zu beschweren, ist es jedoch bevorzugt, den Einsatz der Fettstoffe (d) nach Möglichkeit so gering wie möglich zu halten. Bevorzugt ist es daher, wenn die Gesamtmenge der Fettstoffe (d) im Mittel bei einem Wert unterhalb von 2,0 Gew.-%, bevorzugt unterhalb von 1,5 Gew.-%, weiter bevorzugt unterhalb von 0,5 Gew.-% und besonders bevorzugt unterhalb von 0,1 Gew.-% liegt. Die Gewichtsangaben beziehen sich hierbei auf die Gesamtmenge aller Fettstoffe (d), die zum Gesamtgewicht des Mittels in Relation gesetzt wird.
In einer weiteren ganz besonders bevorzugten Ausführungsform ist ein erfindungsgemäßes Mittel daher dadurch gekennzeichnet, dass die Gesamtmenge aller im Mittel enthaltenen Fettstoffe (d), insbesondere der Fettstoffe aus der Gruppe der C₁₂-C₃₀-Fettalkohole, der C₁₂-C₃₀-Fettsäure-triglyceride, C₁₂-C₃₀-Fettsäurediglyceride, der C₁₂-C₃₀-Fettsäuremonoglyceride, der Ethylenglycol-Difettsäureester, der Wachse und der Kohlenwasserstoffe bei einem Wert unterhalb von 2,0 Gew.-%, bevorzugt unterhalb von 1,5 Gew.-%, weiter bevorzugt unterhalb von 0,5 Gew.-% und besonders bevorzugt unterhalb von 0,1 Gew.-% liegt.

Die Mittel werden als wässrig-alkoholische Zubereitungen bereitgestellt. Die erfindungsgemäßen Silikone (c) sind polyalkoxyliert und besitzen auch emulgierende Eigenschaften. Gegebenenfalls kann den Mitteln zusätzlich eine weitere oberflächenaktive Substanz zugesetzt werden, wobei solche oberflächenaktive Substanzen je nach Anwendungsgebiet als Tenside oder als Emulgatoren bezeichnet werden. Bevorzugt enthalten die erfindungsgemäßen Mittel zusätzlich mindestens ein nichtionisches Tensid und/oder ein kationisches Tensid. Als wenig vorteilhaft hat sich der Einsatz von anionischen Tensiden herausgestellt.
Die erfindungsgemäßen Mittel können zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden.
Die nichtionischen Tenside werden in Mengen von 0,1 bis 45 Gew.%, bevorzugt 1 bis 30 Gew.% und ganz besonders bevorzugt von 1 bis 15 Gew.% - bezogen auf das Gesamtgewicht des Mittels - eingesetzt.
Die erfindungsgemäßen Mittel können zusätzlich mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind.

### Beispiele für Kationtenside sind

- quartäre Ammniumverbindungen, die als hydrophobe Reste ein oder zwei Alkylketten mit einer Kettenlänge von 8 bis 28 C-Atomen tragen können
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein.

Neben der funktionellen Einheit, welche die kationische Ladung trägt, kann das Kationtensid auch weitere ungeladene funktionelle Gruppen beinhalten, dies ist beispielsweise bei Esterquats der Fall. Die kationischen Tenside werden in Mengen von 0,1 bis 45 Gew.%, bevorzugt 1 bis 30 Gew.% und ganz besonders bevorzugt von 1 bis 15 Gew.% - bezogen auf das Gesamtgewicht des Mittels - eingesetzt.

Der Einsatz von anionischen Tensiden hat sich im Hinblick auf die Abriebfestigkeit der Pigmente auf den Keratinfasern als negativ herausgestellt. Aus diesem Grund ist es bevorzugt, in den erfindungsgemäßen Mitteln keine anionischen Tenside einzusetzen.

Als anionische Tenside werden oberflächenaktive Mittel mit ausschließlich anionischen Ladungen (neutralisiert durch ein entsprechendes Gegenkation) bezeichnet.

Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

In einer weiteren bevorzugten Ausführungsform sind erfindungsgemäße Mittel dadurch gekennzeichnet, dass die Gesamtmenge aller im Mittel enthaltenen anionischen Tenside bei einem Wert unterhalb von 2,5 Gew.-%, bevorzugt unterhalb von 1,5 Gew.-%, weiter bevorzugt unterhalb von 0,5 Gew.-% und besonders bevorzugt unterhalb von 0,1 Gew.-% liegt-wobei alle Mengenangaben auf das Gesamtgewicht des Mittels bezogen sind.

Die erfindungsgemäßen Mittel können weiterhin mindestens ein zwitterionisches und/oder amphoteres Tensid enthalten.

Geeignete zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Die amphotern und/oder zwitterionischen Tenside werden in Mengen von 0,1 bis 45 Gew.%, bevorzugt 1 bis 30 Gew.% und ganz besonders bevorzugt von 1 bis 15 Gew.% - bezogen auf das Gesamtgewicht des Mittels - eingesetzt.

Ein Vorteil der erfindungsgemäßen Mittel besteht darin, dass sie sich in vielfältiger Form konfektionieren lassen. Bei Applikation über einen Schwamm oder über eine kleine Bürste können sehr gleichmäßige Farbeffekte und reibechte Färbungen erzielt werden. Ebenso ist es jedoch auch möglich, die erfindungsgemäßen Mittel als Spray zu konfektionieren. Insbesondere auch die durch die Spray-Applikation erhaltenen Färbungen zeichnen sich durch eine sehr hohe Gleichmäßigkeit aus.

Abhängig von der gewählten Applikationsform werden die erfindungsgemäßen Mittel auf eine bestimmte Viskosität eingestellt. Dies erfolgt üblicherweise durch den Einsatz eines oder mehrerer Verdicker. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Besonders einfach und reproduzierbar lässt sich die Viskosität der Mittel durch Polysaccharide, insbesondere Polysaccharide aus der Gruppe der Carboxy-C1-C6-alkyl-cellulosen, der Hydroxy-C2-C8-alkylcellulosen, der Alginsäuren und/oder Xanthan Gum, einstellen.

Durch Variation der eingesetzten Polysaccharidmenge kann das Mittel sowohl als Gel für die Bürsten- bzw. Schwammapplikation oder aber auch als niedrigviskose, sprühbare Lösung konfektioniert werden. Die anderen Rezepturbestandteile bzw. ihre Einsatzmengen müssen hierbei nicht angepasst werden. Dies ist insbesondere bei der Produktion der Mittel von Vorteil.

In einer weiteren besonders bevorzugten Ausführungsform ist ein erfindungsgemäßes Mittel daher dadurch gekennzeichnet, dass es als Verdicker zusätzlich mindestens ein Polysaccharid aus der Gruppe der Carboxy-C₁-C₆-alkyl-cellulosen, der Hydroxy-C₂-C₈-alkylcellulosen, der Alginsäuren und/oder Xanthan Gum enthält.

In einer ganz besonders bevorzugten Ausführungsform ist ein erfindungsgemäßes Mittel dadurch gekennzeichnet, dass es als Verdicker zusätzlich mindestens ein Polysaccharid aus der Gruppe der der Hydroxy-C₂-C₈-alkylcellulosen enthält.

Der oder die Verdicker können in den erfindungsgemäßen Mitteln in einer Gesamtmenge von 0,1 bis 4,5 Gew.-%, bevorzugt von 0,15 bis 3,5 Gew.-% und besonders bevorzugt von 0,2 bis 2,0 Gew.-% - bezogen auf das Gesamtgewicht des Mittels - eingesetzt werden.

Zur Einstellung des pH-Wertes können die erfindungsgemäßen Mittel ein oder mehrere Alkalisierungsmittel enthalten. Die zur Einstellung der gewünschten pH-Werte erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Zur Einstellung des pH-Wertes können die erfindungsgemäßen Mittel ein oder mehrere Säuren aus enthalten. Geeignete Säuren sind beisielsweise organische Säuren wie alpha-Hydroxycarbonsäuren oder anorganische Säuren.

Weiterhin können die Mittel ein oder mehrere nichtionische Polymere enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nichtionische Polymere.
- Stärke und deren Derivate, insbesondere Stärkeether, beispielsweise Structure® XL (National Starch), eine multifunktionelle, salztolerante Stärke;
- Schellack
- Polyvinylpyrrolidone, wie sie beispielsweise unter der Bezeichnung Luviskol® (BASF) vertrieben werden.

Weiterhin können die Mittel (V) und/oder (F) ein oder mehrere Polymere aus der Gruppe Polyquaternium-1, Polyquaternium-2, Polyquaternium-3, Polyquaternium-4, Polyquatenrium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquarternium-64, Polyquaternium-67, Polyquaternium-68, Polyquaternium-69 und/oder Polyquaternium-86 enthalten.

Ferner können die erfindungsgemäßen Mittel weitere Wirk-, Hilfs- und Zusatzstoffe enthalten, wie beispielsweise lineare kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere sowie PEG-3-distearat; Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der jeweiligen Mittel, eingesetzt.

Die erfindungsgemäßen Produkte können beispielsweise in Form eines Gels, eines Sprays, eines Aerosols oder eines Pumpschaums konfektioniert werden. Je nach Anwendungsform werden sie daher bevorzugt in einer Tube, einem Container, einer Flasche, einer Dose, einem Druckbehälter oder in einem Behälter mit Pump-Spray Applikator abgefüllt.

Wenn die Produkte in Spray-Form appliziert werden, lassen sich die Pigmente besonders gleichmäßig auf die Keratinfasern auftragen. Die Konfektionierung als Aerosol oder als Pump-Spray ist daher ganz besonders bevorzugt.

In der vorgenannten bevorzugten Ausführungsform umfasst das erfindungsgemäße Produkt einen Druckbehälter. Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke können die Korrosionsbeständigkeit gegenüber der im Druckbehälter enthaltenen Zubereitung gewährleisten.

Wenn das erfindungsgemäße Produkt über einen Druckbehälter appliziert wird, enthalten die Mittel zusätzlich mindestens ein Treibgas aus der Gruppe Propan, Propen, n-Butan, iso-Butan, iso-Buten, n-Pentan, Penten, iso-Pentan, iso-Penten, Luft, Stickstoff, Argon, N₂O und/oder CO₂.

Innerhalb dieser Gruppe sind die permanenten Gase Luft, Stickstoff, Argon, N₂O und/oder CO₂ bevorzugt, ganz besonders bevorzugt sind Stickstoff, Argon und/oder CO₂.

Weiterhin können die erfindungsgemäßen Mittel auch in Form eines Pump-Sprays zur Anwendung gebracht werden. Geeignete Behälter mit Pumpe oder Squeeze-Mechanismus sind beispielsweise im Handel erhältlich von der Firma Rexam SMT oder Seaquist.

Bei der Anwendung in Form eines Pump-Sprays oder in Form eines Aerosol-Sprays kann der Anwender die erfindungsgemäßen Mittel direkt auf das trockene Haar aufsprühen und auf diesem Wege die gewünschte temporäre Farbänderung erzeugen.

Hierbei kann der Anwender zunächst - beispielsweise durch Kämmen, Toupieren, oder durch die Anwendung eines Lockenstabs - seine Frisur in Form bringen und dann das erfindungsgemäße Mittel aufsprühen. Ebenfalls möglich ist es, zunächst das erfindungsgemäße Mittel aufzusprühen und danach oder währenddessen die Frisur durch die vorgenannten Methoden in Form zu bringen. Ein zweiter Gegenstand der vorliegenden Erfindung ist daher ein Verfahren zur temporären Farb- und Formveränderung von Haaren, worin ein Mittel des ersten Erfindungsgegenstandes, welches in Form eines Pump-Sprays oder Aerosol-Sprays konfektioniert ist, auf die trockenen Haare gesprüht wird und das Haar vor oder während der Applikation zur Frisur gelegt wird.

Bezüglich weiterer bevorzugter Ausführungsformen des erfindungsgemäßen Verfahrens gilt mutatis mutandis das zu den erfindungsgemäßen Mitteln Gesagte.

### Beispiele

Es wurden die folgenden Formulierungen hergestellt - alle Angaben erfolgen, sofern nicht anders angegeben, in Gewichtsprozent (Aktivsubstanz).

**1. Schwamm Applikation**

| | Gew.-% |
|---|---|
| Stearamidopropyldimethylamin | 1,3 |
| Milchsäure | 0,3 |
| Cetyl PEG/PPG-10/1 Dimethicon | 2,0 |
| Glycerin | 2,0 |
| Cetyltrimethylammoniumchlorid | 0,45 |
| PEG-7 Glyceryl Cocoat | 2,0 |
| Hydroxyethylcellulose | 0,2 |
| Colorona Precious Gold (Merck, Mica, CI 77891 (Titanium dioxide), Silica, CI 77491 (Iron oxides), Tin oxide) | 8,5 |
| Ethanol | 51 |
| Wasser | ad 100 |

Die Formulierung wurde mittels eines Schwämmchens auf eine trockene Haarsträhne (Kerling dunkelblond) aufgetragen. Es wurde eine gleichmäßig gefärbte Strähne mit goldenem Schimmer erhalten.

**2. Bürsten Applikation**

| | Gew.-% |
|---|---|
| Stearamidopropyldimethylamin | 1,3 |
| Milchsäure | 0,3 |
| Cetyl PEG/PPG-10/1 Dimethicon | 2,0 |
| Glycerin | 2,0 |
| Cetyltrimethylammoniumchlorid | 0,45 |
| PEG-7 Glyceryl Cocoat | 2,0 |
| Hydroxyethylcellulose | 2,0 |
| Colorona Precious Gold (Merck, Mica, CI 77891 (Titanium dioxide), Silica, CI 77491 (Iron oxides), Tin oxide) | 8,5 |
| Ethanol | 51 |
| Wasser | ad 100 |

Die Formulierung wurde mit einer kleinen Bürste auf eine trockene Haarsträhne (Kerling dunkelblond) aufgetragen. Es wurde eine gleichmäßig gefärbte Strähne mit goldenem Schimmer erhalten.

**3. Spray Applikation**

| | Gew.-% |
|---|---|
| Stearamidopropyldimethylamin | 1,3 |
| Milchsäure | 0,3 |
| Cetyl PEG/PPG-10/1 Dimethicon | 2,0 |
| Glycerin | 2,0 |
| Cetyltrimethylammoniumchlorid | 0,45 |
| PEG-7 Glyceryl Cocoat | 2,0 |
| Colorona Precious Gold (Merck, Mica, CI 77891 (Titanium dioxide), Silica, CI 77491(Iron oxides), Tin oxide) | 8,5 |
| Ethanol | 51 |
| Wasser | ad 100 |

Die Formulierung wurde in einen Pump-Spray Zerstäuber eingefüllt und auf eine trockene Haarsträhne (Kerling dunkelblond) aufgesprüht. Es wurde eine gleichmäßig gefärbte Strähne mit goldenem Schimmer erhalten.

**4. Bestimmung der Pigmentverteilung in Formulierungen zur Spray Applikation**

| | V1 | V2 | E |
|---|---|---|---|
| Stearamidopropyldimethylamin | 1,3 | 1,3 | 1,3 |
| Milchsäure | 0,3 | 0,3 | 0,3 |
| Cyclomethicon (85 Gew.-%), Dimethiconol (15 Gew.-%) | 2,0 | --- | --- |
| Bis-Cetearyl Amodimethicone | --- | 2,0 | --- |
| Cetyl PEG/PPG-10/1 Dimethicon | --- | --- | 2,0 |
| Glycerin | 2,0 | 2,0 | 2,0 |
| Cetyltrimethylammoniumchlorid | 0,45 | 0,45 | 0,45 |
| PEG-7 Glyceryl Cocoat | 2,0 | 2,0 | 2,0 |
| Colorona Precious Gold (Merck, Mica, CI 77891 (Titanium dioxide), Silica, CI 77491 (Iron oxides), Tin oxide) | 8,5 | 8,5 | 8,5 |
| Ethanol | 51 | 51 | 51 |
| Wasser | ad 100 | ad 100 | ad 100 |

Die Formulierungen V1, V2 und E wurden in einen Pump-Spray Zerstäuber gefüllt. Kurz vor der Anwendung wurde der Pump-Spray Zerstäuber kurz geschüttelt, dann wurde jede Formulierung auf eine Haarsträhne aufgesprüht.

| V1 | V2 | V3 |
|---|---|---|
| Farbpigmente (Colorona Precoius Gold) ballten sich zusammen, Formulierung nicht sprühbar | Farbpigmente (Colorona Precoius Gold) ballten sich zusammen, Formulierung nicht sprühbar | feindisperse Formulierung, gut sprühbar, homogenes Farbergebnis mit guter Reibechtheit |

## Patentansprüche

1. Mittel zur temporären Farbveränderung von keratinischen Fasern, insbesondere menschlichen Haaren, enthaltend in einem wässrigen kosmetischen Träger - bezogen auf das Gesamtgewicht des Mittels -
(a) einen oder mehrere aliphatische und/oder aromatische Alkohole mit 2 bis 8 C-Atomen in einer Gesamtmenge von mindestens 35,0 Gew.-%,
(b) mindestens ein Farbpigment, das bei 20 °C in Wasser eine Löslichkeit von weniger als 0,1 g/l besitzt, und
(c) mindestens ein nichtionisches, polyalkoxyliertes Silikon, welches mindestens eine Struktureinheit der allgemeinen Formel (II) umfasst, 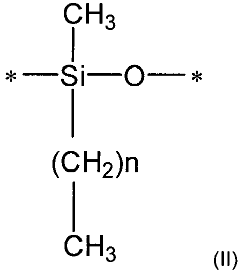 wobei
n für eine ganze Zahl von 8 bis 40 steht, und wobei
- die Gesamtmenge der im Mittel enthaltenen Fettstoffe (d) aus der Gruppe der C₁₂-C₃₀-Fettalkohole, der C₁₂-C₃₀-Fettsäuretriglyceride, C₁₂-C₃₀-Fettsäurediglyceride, der C₁₂-C₃₀-Fettsäuremonoglyceride, der Ethylenglycol-Difettsäureester, der Wachse und der Kohlenwasserstoffe bei einem Wert unterhalb von 2,5 Gew.-% liegt, und
- das Mittel mindestens 30,0 Gew.-% Ethanol enthält.

2. Mittel nach Anspruch 1, **dadurch gekennzeichnet, dass** es einen oder mehrere Alkohole (a) aus der Gruppe Ethanol, Isopropanol, n-Propanol, Butanol, n-Pentanol, 1,2-Propandiol, 1,3-Propandiol, 1,3-Butandiol, 1,4-Butandiol, 1,2-Hexandiol, 1,6-Hexandiol, Glycerin, Benzylalkohol, Phenoxyethanol und Phenylethylalkohol enthält.

3. Mittel nach einem der Ansprüche 1 bis 2, **dadurch gekennzeichnet, dass** es - bezogen auf sein Gesamtgewicht - einen oder mehrere Alkohole (a) in einer Gesamtmenge von mindestens 40,0 Gew.-% und ganz besonders bevorzugt von mindestens 50,0 Gew.-% enthält.

4. Mittel nach einem der Ansprüche 1 bis 3, **dadurch gekennzeichnet, dass** es - bezogen auf sein Gesamtgewicht - mindestens 35,0 Gew.-%, weiter bevorzugt mindestens 40,0 Gew.-% und ganz besonders bevorzugt mindestens 45,0 Gew.-% Ethanol enthält.

5. Mittel nach einem der Ansprüche 1 bis 4, **dadurch gekennzeichnet, dass** es - bezogen auf sein Gesamtgewicht - 1,2-Propandiol und/oder Glycerin in einer Gesamtmenge von 0,1 bis 7,0 Gew.-%, bevorzugt 0,5 bis 5,5 Gew.-%, weiter bevorzugt 1,0 bis 3,5 Gew.-% und besonders bevorzugt 1,5 Gew.-% bis 2,5 Gew.-% enthält.

6. Mittel nach einem der Ansprüche 1 bis 5, **dadurch gekennzeichnet, dass** es - bezogen auf sein Gesamtgewicht - einen Wassergehalt zwischen 20 und 60 Gew.-%, bevorzugt zwischen 24 und 54 Gew.-%, weiter bevorzugt zwischen 28 und 50 Gew.-% und besonders bevorzugt zwischen 32 und 42 Gew.-% besitzt.

7. Mittel nach einem der Ansprüche 1 bis 6, **dadurch gekennzeichnet, dass** es als Farbpigment (b) mindestens ein anorganisches Farbpigment enthält, welches ausgewählt ist aus farbigen Metalloxiden, Metallhydroxiden, Metalloxidhydraten, Silicaten, Metallsufiden, komplexen Metallcyaniden, Metallsulfaten, Bronzepigmenten und/oder aus farbigen Pigmenten auf Mica- oder Glimmerbasis, die mit mindestens einem Metalloxid und/oder einem Metalloxychlorid beschichtet sind.

8. Mittel nach einem der Ansprüche 1 bis 7, **dadurch gekennzeichnet, dass** es als Farbpigment (b) mindestens ein farbiges Pigment auf Mica- oder Glimmerbasis enthält, das mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet ist.

9. Mittel nach einem der Ansprüche 1 bis 8, **dadurch gekennzeichnet, dass** es - bezogen auf sein Gesamtgewicht - ein oder mehrere Farbpigmente (b) in einer Gesamtmenge von 1,0 bis 25,0 Gew-%, bevorzugt von 5,0 bis 20,0 Gew.-%, weiter bevorzugt von 7,0 bis 18,0 Gew.-% und besonders bevorzugt von 8,5 bis 15,5 Gew.-% enthält.

10. Mittel nach einem der Ansprüche 1 bis 9, **dadurch gekennzeichnet, dass** es ein nichtionisches, polyalkoxyliertes Silikon (c) enthält, welches mindestens eine Struktureinheit der allgemeinen Formel (I) umfasst, 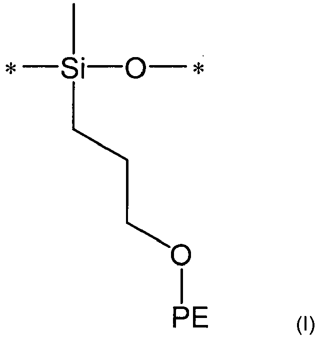 wobei
PE gleich für eine Gruppierung -(C₂H₄O)ₓ-(C₃H₆O)_{y}-H oder für eine Gruppierung -(C₃H₆O)_{y}-(C₂H₄O)ₓ-H steht,
x für eine ganze Zahl von 0 bis 20 steht,
y für eine ganze Zahl von 0 bis 20 steht und
die Summe aus x und y mindestens 2 beträgt.

11. Mittel nach einem der Ansprüche 1 bis 10, **dadurch gekennzeichnet, dass** es ein nichtionisches, polyalkoxyliertes Silikon (c) enthält, welches mindestens eine Struktureinheit der allgemeinen Formel (II) umfasst, 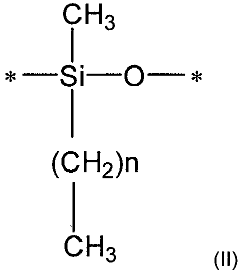 wobei
n für eine ganze Zahl von 10 bis 30 und besonders bevorzugt von 14 bis 20 steht.

12. Mittel nach einem der Ansprüche 1 bis 11, **dadurch gekennzeichnet, dass** es als nichtionisches, polyalkoxyliertes Silikon (c) Cetyl PEG/PPG-10/1 Dimethicon enthält.

13. Mittel nach einem der Ansprüche 1 bis 12, **dadurch gekennzeichnet, dass** es - bezogen auf sein Gesamtgewicht - ein oder mehrere nichtionische, polyalkoxylierte Silikone (c) in einer Gesamtmenge von 0,1 bis 10,0 Gew-%, bevorzugt von 0,5 bis 5,0 Gew.-%, weiter bevorzugt von 0,8 bis 3,0 Gew.-% und besonders bevorzugt von 1,4 bis 2,6 Gew.-% enthält.

14. Mittel nach einem der Ansprüche 1 bis 13, **dadurch gekennzeichnet, dass** das Gewichtsverhältnis aus allen im Mittel enthaltenden Farbpigmenten (b) zu allen im Mittel enthaltenen nichtionischen, polyalkoxylierten Silikonen (c), d.h. das Gewichtsverhältnis (b)/(c), bei einem Wert von 1,0 bis 6,0, bevorzugt von 2,0 bis 5,5, weiter bevorzugt von 2,5 bis 5,0 und besonders bevorzugt von 3,0 bis 4,5 liegt.

15. Verfahren zur temporären Farb- und Formveränderung von Haaren, worin ein Mittel nach einem der Ansprüche 1 bis 14, welches in Form eines Pump-Sprays oder Aerosol-Sprays konfektioniert ist, auf die trockenen Haare gesprüht wird und das Haar vor oder während der Applikation zur Frisur gelegt wird.

## Claims

1. An agent for temporarily changing the color of keratin fibers, in particular human hair, containing in an aqueous cosmetic carrier, based on the total weight of the agent,
(a) one or more aliphatic and/or aromatic alcohols having 2 to 8 C atoms in a total amount of at least 35.0 wt.%,
(b) at least one pigment which has a solubility in water at 20 °C of less than 0.1 g/l, and
(c) at least one nonionic polyalkoxylated silicone which comprises at least one structural unit of general formula (II), 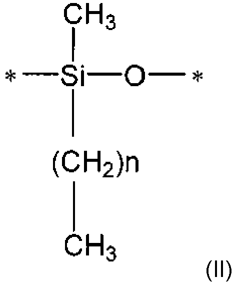 where
n represents an integer from 8 to 40, and wherein
- the total amount of fatty substances (d) contained in the agent from the group of C₁₂-C30 fatty alcohols, C12-C30 fatty acid triglycerides, C12-C30 fatty acid diglycerides, C12-C30 fatty acid monoglycerides, ethylene glycol di-fatty acid esters, waxes and hydrocarbons has a value below 2.5 wt.%, and
- the agent contains at least 30.0 wt.% ethanol.

2. The agent according to claim 1, **characterized in that** it contains one or more alcohols (a) from the group ethanol, isopropanol, n-propanol, butanol, n-pentanol, 1,2-propanediol, 1,3-propanediol, 1,3-butanediol, 1,4-butanediol, 1,2-hexanediol, 1,6-hexanediol, glycerol, benzyl alcohol, phenoxyethanol and phenylethyl alcohol.

3. The agent according to one of claims 1 to 2, **characterized in that** it contains, based on the total weight thereof, one or more alcohols (a) in a total amount of at least 40.0 wt.%, and very particularly preferably of at least 50.0 wt.%.

4. The agent according to one of claims 1 to 3, **characterized in that** it contains, based on the total weight thereof, at least 35.0 wt.%, more preferably at least 40.0 wt.%, and very particularly preferably at least 45.0 wt.%, of ethanol.

5. The agent according to one of claims 1 to 4, **characterized in that** it contains, based on the total weight thereof, 1,2-propanediol and/or glycerol in a total amount of from 0.1 to 7.0 wt.%, preferably from 0.5 to 5.5 wt.%, more preferably from 1.0 to 3.5 wt.%, and particularly preferably from 1.5 to 2.5 wt.%.

6. The agent according to one of claims 1 to 5, **characterized in that** it contains, based on the total weight thereof, a water content between 20 and 60 wt.%, preferably between 24 and 54 wt.%, more preferably between 28 and 50 wt.% and particularly preferably between 32 and 42 wt.%.

7. The agent according to one of claims 1 to 6, **characterized in that** it contains, as pigment (b), at least one inorganic pigment which is selected from colored metal oxides, metal hydroxides, metal oxide hydrates, silicates, metal sulfides, complex metal cyanides, metal sulfates, bronze pigments and/or from mica-based colored pigments which are coated with at least one metal oxide and/or a metal oxychloride.

8. The agent according to one of claims 1 to 7, **characterized in that** it contains, as pigment (b), at least one mica-based colored pigment which is coated with one or more metal oxides from the group of titanium dioxide (CI 77891), black iron oxide (CI 77499), yellow iron oxide (CI 77492), red and/or brown iron oxide (CI 77491, CI 77499), manganese violet (CI 77742), ultramarine (sodium aluminum sulfosilicates, CI 77007, pigment blue 29), chromium oxide hydrate (CI 77289), chromium oxide (CI 77288) and/or iron blue (ferric ferrocyanide, CI 77510).

9. The agent according to one of claims 1 to 8, **characterized in that** it contains, based on the total weight thereof, one or more pigments (b) in a total amount of from 1.0 to 25.0 wt.%, preferably from 5.0 to 20.0 wt.%, more preferably from 7.0 to 18.0 wt.%, and particularly preferably from 8.5 to 15.5 wt.%.

10. The agent according to one of claims 1 to 9, **characterized in that** it contains a nonionic polyalkoxylated silicone (c) which comprises at least one structural unit of general formula (I), 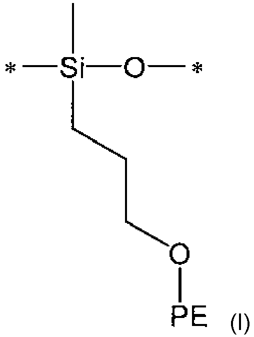 where
PE equally represents a -(C₂H₄O)ₓ-(C₃H₆O)_{y}-H grouping or a -(C₃H₆O)_{y}-(C₂H₄O)ₓ-H grouping,
x represents an integer from 0 to 20,
y represents an integer from 0 to 20, and
the sum of x and y is at least 2.

11. The agent according to one of claims 1 to 10, **characterized in that** it contains a nonionic polyalkoxylated silicone (c) which comprises at least one structural unit of general formula (II), 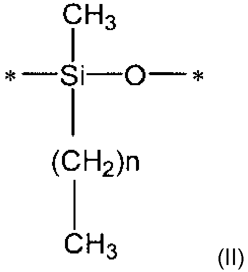 where
n represents an integer from 10 to 30 and particularly preferably from 14 to 20.

12. The agent according to one of claims 1 to 11, **characterized in that** it contains cetyl PEG/PPG-10/1 dimethicone as the nonionic polyalkoxylated silicone (c).

13. The agent according to one of claims 1 to 12, **characterized in that** it contains, based on the total weight thereof, one or more nonionic polyalkoxylated silicones (c) in a total amount of from 0.1 to 10.0 wt.%, preferably from 0.5 to 5.0 wt.%, more preferably from 0.8 to 3.0 wt.%, and particularly preferably from 1.4 to 2.6 wt.%.

14. The agent according to one of claims 1 to 13, **characterized in that** the weight ratio of all pigments (b) contained in the agent to all nonionic polyalkoxylated silicones (c) contained in the agent, i.e. the weight ratio (b)/(c), has a value of from 1.0 to 6.0, preferably from 2.0 to 5.5, more preferably from 2.5 to 5.0 and particularly preferably from 3.0 to 4.5.

15. A method for temporarily changing the color and shape of hair, in which an agent according to one of claims 1 to 14 which is packaged in the form of a pump spray or aerosol spray is sprayed onto the dry hair and the hair is styled before or during application.

## Revendications

1. Agent pour le changement de couleur temporaire des fibres kératiniques, en particulier des cheveux humains, contenant, dans un support cosmétique aqueux - par rapport au poids total de l'agent -
(a) un ou plusieurs alcools aliphatiques et/ou aromatiques ayant de 2 à 8 atomes de carbone en une quantité totale d'au moins 35,0 % en poids,
(b) au moins un pigment de couleur ayant une solubilité inférieure à 0,1 g/l à 20 °C dans l'eau, et
(c) au moins une silicone polyalcoxylée non ionique, qui comprend au moins une unité de structure de la formule générale (II), 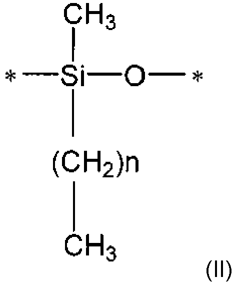 dans laquelle
n représente un nombre entier compris entre 8 et 40, et
- la quantité totale de corps gras (d) contenus dans l'agent choisis dans le groupe des alcools gras en C₁₂ à C₃₀, des triglycérides d'acides gras en C₁₂ à C₃₀, des diglycérides d'acides gras en C₁₂ à C₃₀, des esters d'acides gras en C₁₂ à C₃₀, des cires et des hydrocarbures étant inférieure à 2,5 % en poids, et
- l'agent contenant au moins 30,0 % en poids d'éthanol.

2. Agent selon la revendication 1, **caractérisé en ce qu'**il contient un ou plusieurs alcools (a) choisis dans le groupe constitué de l'éthanol, de l'isopropanol, du n-propanol, du butanol, du n-pentanol, du 1,2-propanediol, du 1,3-propanediol, du 1,3-butanediol, du 1,4-butanediol, du 1,2-hexanediol, du 1,6-hexanediol, du glycérol, de l'alcool benzylique, du phénoxyéthanol et de l'alcool phényléthylique.

3. Agent selon l'une quelconque des revendications 1 à 2, **caractérisé en ce qu'**il contient - par rapport à son poids total - un ou plusieurs alcools (a) en une quantité totale d'au moins 40,0 % en poids et de manière tout particulièrement préférée d'au moins 50,0 % en poids.

4. Agent selon l'une quelconque des revendications 1 à 3, **caractérisé en ce qu'**il contient - par rapport à son poids total - au moins 35,0 % en poids, de manière davantage préférée au moins 40,0 % en poids et de manière tout particulièrement préférée au moins 45,0 % en poids d'éthanol.

5. Agent selon l'une quelconque des revendications 1 à 4, **caractérisé en ce qu'**il contient - par rapport à son poids total - du 1,2-propanediol et/ou du glycérol en une quantité totale de 0,1 à 7,0 % en poids, de préférence de 0,5 à 5,5 % en poids, de manière davantage préférée de 1,0 à 3,5 % en poids et de manière particulièrement préférée de 1,5 à 2,5 % en poids.

6. Agent selon l'une quelconque des revendications 1 à 5, **caractérisé en ce qu'**il possède - par rapport à son poids total - une teneur en eau comprise entre 20 et 60 % en poids, de préférence entre 24 et 54 % en poids, de manière davantage préférée entre 28 et 50 % en poids et de manière particulièrement préférée entre 32 et 42 % en poids.

7. Agent selon l'une quelconque des revendications 1 à 6, **caractérisé en ce qu'**il contient, en tant que pigment de couleur (b), au moins un pigment de couleur inorganique choisi parmi les oxydes métalliques, les hydroxydes métalliques, les hydrates d'oxydes métalliques, les silicates, les sulfures métalliques, les cyanures métalliques complexes, les sulfates métalliques, les pigments de couleur bronze et/ou parmi les pigments de couleur à base de mica revêtus d'au moins un oxyde métallique et/ou d'un oxychlorure métallique.

8. Agent selon l'une quelconque des revendications 1 à 7, **caractérisé en ce qu'**il contient, en tant que pigment de couleur (b), au moins un pigment coloré à base de mica, qui est recouvert d'un ou de plusieurs oxydes métalliques du groupe constitué du dioxyde de titane (CI 77891), de l'oxyde de fer noir (CI 77499), de l'oxyde de fer jaune (CI 77492), de l'oxyde de fer rouge et/ou brun (CI 77491, CI 77499), du violet de manganèse (CI 77742), de l'ultramarine (sulfosilicates de sodium et d'aluminium, CI 77007, pigment de couleur Blue 29), de l'oxyde de chrome hydraté (CI 77289), de l'oxyde de chrome (CI 77288) et/ou du bleu de fer (ferrocyanure ferrique, CI 77510).

9. Agent selon l'une quelconque des revendications 1 à 8, **caractérisé en ce qu'**il contient - par rapport à son poids total - un ou plusieurs pigments de couleur (b) en une quantité totale de 1,0 à 25,0 % en poids, de préférence de 5,0 à 20,0 % en poids, de manière davantage préférée de 7,0 à 18,0 % en poids et de manière particulièrement préférée de 8,5 à 15,5 % en poids.

10. Agent selon l'une quelconque des revendications 1 à 9, **caractérisé en ce qu'**il contient une silicone polyalcoxylée non ionique (c) comprenant au moins une unité de structure de la formule générale (I), 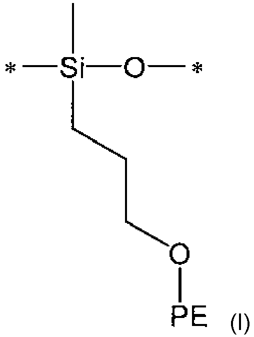 dans laquelle
PE est identique pour un groupement - (C₂H₄O)ₓ - (C₃H₆O)_{y}-H, ou pour un groupement -(C₃H₆O)_{y}-(C₂H₄O)ₓ-H,
x représente un nombre entier compris entre 0 et 20,
y représente un nombre entier compris entre 0 et 20 et
la somme de x et de y est au moins de 2.

11. Agent selon l'une quelconque des revendications 1 à 10, **caractérisé en ce qu'**il contient une silicone polyalcoxylée non ionique (c) comprenant au moins une unité de structure de la formule générale (II), 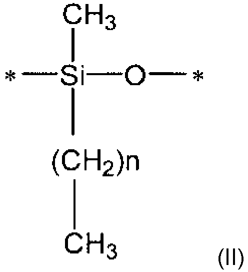 dans laquelle
n est un entier de 10 à 30 et de manière particulièrement préférée de 14 à 20.

12. Agent selon l'une quelconque des revendications 1 à 11, **caractérisée en ce qu'**il contient, comme silicone polyalkoxylée non ionique (c), du cétyl-PEG/PPG-10/1-diméthicone.

13. Agent selon l'une quelconque des revendications 1 à 12, **caractérisé en ce qu'**il contient - par rapport à son poids total - une ou plusieurs silicones polyalkoxylées non ioniques (c) en une quantité totale de 0,1 à 10,0 % en poids, de préférence de 0,5 à 5,0 % en poids, de manière davantage préférée de 0,8 à 3,0 % en poids et de manière particulièrement préférée de 1,4 à 2,6 % en poids.

14. Composition selon l'une quelconque des revendications 1 à 13, **caractérisée en ce que** le rapport pondéral entre tous les pigments de couleur (b) contenus dans l'agent et toutes les silicones polyalkoxylées non ioniques (c) contenues dans l'agent, c'est-à-dire le rapport pondéral (b)/(c), s'élève à une valeur de 1,0 à 6,0, de préférence de 2,0 à 5,5, de manière davantage préférée de 2,5 à 5,0 et de manière particulièrement préférée de 3,0 à 4,5.

15. Procédé de changement temporaire de couleur et de forme des cheveux, dans lequel un agent selon l'une quelconque des revendications 1 à 14, qui est confectionné sous la forme d'un spray à pompe ou d'un aérosol de pulvérisation, est pulvérisé sur les cheveux secs et les cheveux sont coiffés avant ou pendant l'application.
